# EUROPEAN PATENT SPECIFICATION

(11) **EP 2 030 582 B1**
(45) Date of publication and mention of the grant of the patent: **30.03.2011**
(21) Application number: 07016859.6
(22) Date of filing: 28.08.2007
(51) Int. Cl.: A61B 17/22, A61B 17/00

(54) **System and method for monitoring the adjustment of a lithotripter and an X-ray C-arc**
System und Verfahren zur Überwachung der Ausrichtung eines Nierensteinzertrümmerers und eines Röntgen-C-Bogens
Système et procédé de surveillance du réglage de lithotriteur et arc de rayon X

(43) Date of publication of application: 04.03.2009
(73) Proprietor: Dornier MedTech Systems GmbH, 82234 Wessling (DE)
(72) Inventor: Buchbauer, Peter, Dipl.-Ing., 85748 Garching (DE); Grözinger, Reiner, 82239 Alling (DE); Pötting, Kay, 82282 Wenigmünchen (DE); Treutler, Thomas, 82205 Gilching (DE); Weislmeier, Rudolf, 86899 Landsberg (DE)
(74) Representative: Grünecker, Kinkeldey, Stockmair & Schwanhäusser Anwaltssozietät

(56) References cited:
- EP-A- 1 514 520
- DE-A1- 10 032 982
- DE-A1-102005 013 292
- DE-B4- 10 337 519

## Description

The present invention relates to a system and a method for monitoring the adjustment of a lithotripter and an x-ray C-arc with the features of the preamble of claim 1.

DE 103 37 519 B4 describes a modular connectable apparatus arrangement, in which a lithotripter and an x-ray unit are mechanically coupled and thus adjusted relative to each other by coupling components. To monitor the adjustment of the therapy head of the lithotripter and the C-arc of the x-ray unit, the arm carrying the therapy head is provided with two laser pointers. The light beams generated by these laser pointers hit the inner surface of the C-arc. One of the light beams hits the upper half of the C-arc whereas the second light beam hits the lower half of the C-arc, thereby indicating the position of the plane by the carrying arm of the therapy head. By means of the laser pointers, this plane can be aligned with the plane spanned by the C-arc.

However, this monitoring is only possible when the therapy head and the carrying arm of the lithotripter are in predetermined positions, namely only when the therapy head is in the straight over-table position or when it is turned about 180° into the straight under-table position. Furthermore, the above-mentioned system only allows an adjustment of the lithotripter and the x-ray unit before the therapy is started, since during therapy it can be necessary to change the position of the therapy head to a position differing from a straight over- or under-table position. Thus, monitoring of the correct adjustment during therapy is not possible with this system. Moreover, the correct position of the light beam hitting the lower half of the C-arc is often hard to determine, since, depending on the position of the observer, the view on the lower half of the C-arc is often obstructed by several components, like the patient bed, the patient or the carrying arm of the therapy head.

It is therefore the object of the present invention to improve an adjusting system and method of the above-mentioned kind such that it permits a good monitoring of the correct adjustment, also during therapy, and concurrently is easy to handle as well as to survey.

This object is achieved by a system having the features of claim 1.

Since the light emitters of the invention and the generated light beams correspond to different positions of the C-arc, the inventive system allows moving the C-arc between at least two predetermined positions while the correct alignment of the C-arc with the lithotripter can be monitored in each of these positions. This is, for example, necessary in order to locate a concrement in a patient's body by taking x-ray photos in different positions of the C-arc. Thus, it is possible with the inventive system to locate a concrement and, if the patient needs to be moved during therapy, also to relocate a concrement while concurrently monitoring the correct alignment of the lithotripter and the x-ray C-arc.

Advantageously, the different light emitters can correspond to different tilting positions of the C-arc. This allows a good monitoring of the adjustment during location of a concrement.

In addition, each light emitter can be separately hinged, thereby permitting a good adjustment of each of the light emitters.

According to an embodiment of the invention, each light emitter may be hinged by a magnetic ball joint mounting. This allows a good and secure adjustment of the light emitter in every desired position.

Further, the light emitters can be adjustable independently from each other. This permits the adjustment of each of the light emitters to arbitrary positions of the C-arc and thus allows adaptation of the system to different applications.

In an embodiment of the invention, each light emitter can be controlled separately, thereby allowing the switching off of presently unnecessary light emitters not corresponding to the present position of the C-arc. This also reduces the risk of glaring or irritation of medical personnel.

Additionally, the light emitters can be adjustable manually and/or via at least one drive motor. This allows the light emitters to be controlled from a remote place or by hand in case the drive motors should fail.

Moreover, the light emitters can be controllable via a remote control. This permits controlling the light emitters from a remote place, e.g. a separate control room being different from the examination room, thereby protecting the medical personnel from x-rays. Further, it increases the convenience of the inventive monitoring system.

Favourably, the light emitters can be positioned on static parts of the lithotripter. Thus, the therapy head of the lithotripter and the arm carrying the therapy head can be moved during therapy independently from the light emitters.

In addition, the light emitters can be positioned at a height being higher than a maximum height of a support arm carrying a therapy arm of the lithotripter. Thus, the therapy head of the lithotripter can be moved during therapy without interrupting the light beam and the correct alignment of the lithotripter and the C-arc can be constantly monitored.

Advantageously, the light beams generated by the light emitters can be slanted versus the spatial coordinates (X, Y, Z) spanned by the arrangement of the lithotripter and the C-arc. Since unintended movement of the lithotripter and/or the C-arc usually occurs along one of the spatial coordinates, this helps to ensure that every misalignment of the lithotripter and the C-arc is indicated by the light beams.

In another embodiment of the invention, the light beams generated by the light emitters can be slanted versus the floor on which the lithotripter stands. This also helps to visualize any possible misalignment of the lithotripter and the C-arc.

In a further embodiment of the invention, at least three light emitters can be provided, corresponding to three different positions of the C-arc, in particular one light emitter corresponding to a PA position of the C-arc, one to the CC+ and one to the CC- position of the C-arc. This permits the monitoring of three different positions of the C-arc, particularly the most commonly used positions of the C-arc during therapy.

Beneficially, the positioning marker can be located on the upper half of the C-arc. The elevated position of the positioning marker allows a good visibility of the marker and eases the monitoring for the medical personnel.

Further, only one positioning marker can be provided, matching each of the light beams. Thus the monitoring process can be simplified by monitoring just one marker at a time.

In an advantageous embodiment of the invention, the positioning marker can comprise crosshairs. This allows the determination of the direction of the deviation from the correct alignment. By providing a scale on the axis of the crosshairs, it can also permit the determination of a value of the deviation.

Moreover, the positioning marker can comprise a marker, in particular a circle, indicating the tolerance limits of misalignment. This further facilitates monitoring and maintaining of the correct alignment of the lithotripter and the C-arc within the tolerance limits of misalignment.

Additionally, the light beam can hit the centre of the crosshairs when a correct adjustment of the lithotripter to the C-arc is maintained. Thus, the correct alignment of the lithotripter and the C-arc can almost constantly be monitored, even when the therapy head is moved during therapy.

In a favourable embodiment of the invention, a therapy head and a therapy arm of the lithotripter can be moveable independently from the light emitters. Thus, the correct alignment of the lithotripter and the C-arc can even be monitored during movement or adaptation of the therapy head to a different therapy position.

The object of the invention is further attained with the features of claim 19.

This method allows the monitoring of the correct alignment of the lithotripter and the C-arc even if the C-arc takes different positions, that is to say is moved during therapy. This is important e.g. for the location of a concrement in a patient. Also if, for example, the patient is moved during therapy and the concrement has to be relocated to continue therapy this method allows the relocation to be done directly during therapy by moving the C-arc into predetermined positions. During this process, the correct alignment of the lithotripter and the C-arc can be further monitored, thereby ensuring that the correct set up of the lithotripter and C-arc is still being maintained.

Moreover, each light emitter can be adjusted such that the corresponding light beam hits the positioning marker at a predetermined tilting position of the C-arc. This is particularly favourable in locating a concrement in a patient's body.

In a beneficial embodiment of the invention, the light emitters can be adjusted such that the light beam of the first light emitter hits the positioning marker when the C-arc is in PA position and the light beam of the second light emitter hits the positioning marker when the C-arc is in one of CC+ or CC- position. Thus the correct alignment of the lithotripter and the C-arc can be monitored in both positions most commonly used when locating a concrement in a patient's body.

In a further embodiment of the invention, a third light emitter can be adjusted such that a corresponding light beam hits the positioning marker when the C-arc is in the other of the CC+ or CC- positions. This permits the monitoring of the correct alignment in even more than two different positions of the C-arc, which may be helpful in certain applications or when locating a concrement is difficult by only two x-ray pictures.

Favourably, only one light emitter can be energized at one time. This helps to prevent glaring and/or irritating of the medical personnel.

Further, when the C-arc is in a predetermined position, only the light emitter corresponding to this position can be energized. This also helps to prevent irritation of the medical personnel by light beams presently not in use and thus not hitting the positioning marker.

Advantageously, all light emitters can be turned off. This allows to completely switch off the monitoring system and thus helps to prevent glaring of medical personnel, when monitoring the adjustment of the lithotripter and the C-arc is not needed, for example, when the concrement is located by different means from x-ray, e.g. ultrasound only location.

Moreover, all light emitters can be turned on. This permits operating the C-arc by remote control, e.g. the medical personnel being in a separate control room for protection from x-rays.

In another embodiment of the invention, a therapy head of the lithotripter can be moved independently from the light emitters, thus allowing monitoring of the correct alignment of the lithotripter and the C-arc even when the head of the lithotripter is moved during therapy.

Beneficially, in the case of misalignment, the lithotripter and the C-arc can be readjusted by movement of the lithotripter and/or the C-arc such that the light beams generated by the light emitters hit the positioning marker, in particular the center of the crosshairs within a misalignment marker. This allows to quickly correct small extents of misalignment without the need of repeating the whole, time-consuming adjustment procedure.

In the following, an embodiment of the invention is described based on the subsequent drawing.
- Figure 1: shows a lithotripter and a C-arc of an x-ray unit equipped with an embodiment of the inventive monitoring system,
- Figure 2: shows a close view to the light emitters and the positioning marker of the monitoring system,
- Figure 3: shows the inventive system with the C-arc tilted to a CC- position, and
- Figure 4: shows the inventive system with the C-arc tiled to a CC+ position.

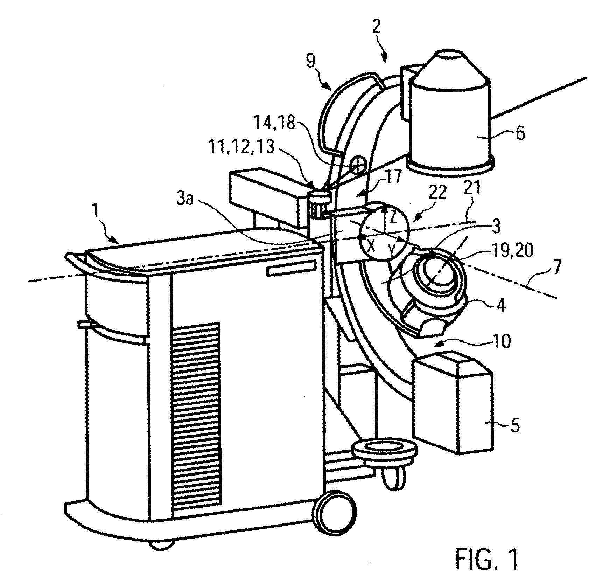

Figure 1 shows a lithotripter 1 and an x-ray C-arc 2 equipped with an embodiment of the inventive monitoring system. The lithotripter 1 has a moveable arm 3 carrying the therapy head 4 mounted on a support arm 3a. The C-arc 2 is provided with an x-ray source 5 and an image intensifier 6 mounted to opposite ends thereof, and is tiltable about a tilting axis 7 by swiveling joint 8. The swiveling joint 8 divides the C-arc 2 into an upper half 9 positioned above the swiveling joint 8 and a lower half 10 positioned between the swiveling joint and the floor. In this embodiment of the invention, the lithotripter 1 is provided with three light emitters 11, 12, 13 and the C-arc 2 is provided with one positioning marker 14 corresponding to the light emitters 11, 12, 13.

Figure 1 shows the lithotripter 1 and the C-arc 2 in a typical arrangement for aligning a focus point 20 of the therapy head 4 with an isocenter 19 of the C-arc 2. In this instance, the lithotripter 1 and the C-arc 2 ideally take a position perpendicular to each other. The C-arc 2 and the curved arm 3 of the lithotripter 1 are arranged approximately concentrically, with the arm 3 being positioned within the C-arc 2. However, the particular design of the C-arc 2 and the lithotripter 1 as such are not important to the invention. Alternatively, other common lithotripters or C-arcs having shapes and design differing from the ones described above can be used with the inventive monitoring system.

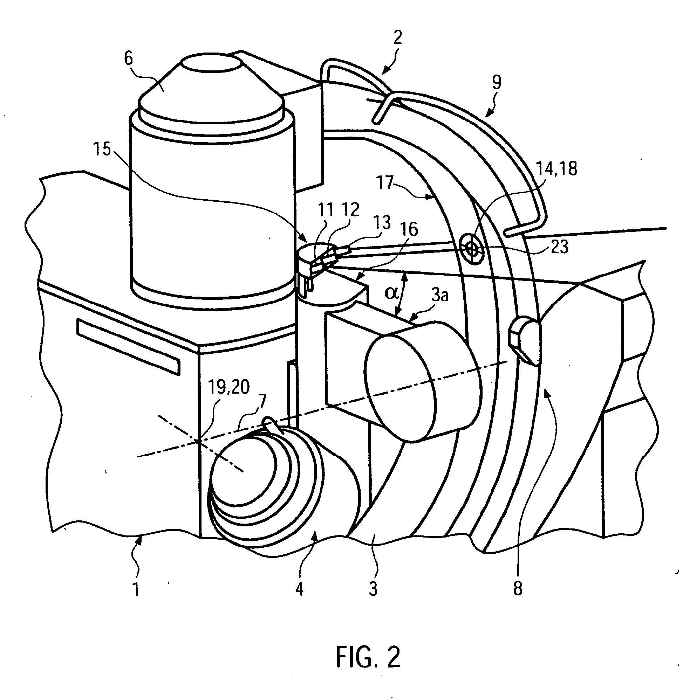

Figure 2 shows a closer view to the light emitters 11, 12, 13 and the positioning marker 14. The reference numerals of Figure 2 indicate the same parts as in Figure 1. In the present embodiment of the invention, the three light emitters 11, 12, 13 are arranged in a holder 15 that is mounted on top of a support 16 for the arm 3. The holder 15 is elevated to an increased height, which is preferably higher than the maximum height of the support arm 3a above the floor and lower than the average height of a human head. This position allows a good visibility of the hitting point of the light beam on the positioning marker 14. Concurrently, this elevated position helps to prevent glaring and irritation of the medical personnel by the light beam.

As can be seen in Figure 2, the light emitters 11, 12, 13 are slightly turned towards the inner surface 17 of the C-arc 2. Thus, a light beam generated by one of the light emitters 11, 12, 13 extends away from the support arm 3a of the lithotripter 1 at a predetermined angle α and towards the C-arc 2. Nevertheless, in a different embodiment of the invention, the light emitters are not necessarily arranged in a holder but can also be mounted directly on the lithotripter.

Each light emitter 11, 12, 13 is separately hinged to the holder 15 and is thus individually adjustable. In the present invention, the light emitters are mounted on the holder 15 via a magnetic ball joint and are positioned such that the light beams generated by them have a slanted direction versus the X, Y and Z axis of the spatial coordinates given by the lithotripter 1 and the C-arc 2. Ideally, the lithotripter 1 and the C-arc 2 are placed perpendicular to each other. In this case, a longitudinal axis (21) of the lithotripter 1 and the tilting axis 7 of the C-arc 2 represent the X and Y axes of this spatial coordinate system (22). The x-ray beam generated by the C-arc 2 would then represent the Z direction of this spatial coordinate system (22). However, in practice, it is sufficient to place the lithotripter 1 and the C-arc 2 only approximately perpendicular relative to each other. The inventive monitoring system will nevertheless work well.

In the magnetic ball joint, either the socket or the ball is a solenoid while the other one is made of a magnetic material like steel. Due to the attractive force and the friction between these two joint elements, a light emitter 11, 12, 13 can be fixed in every position the ball joint allows it to take. Alternatively, the light emitters 11, 12, 13 can be mounted on the holder 15 or directly on the lithotripter 1 by different joining elements, like a conventional ball and socket joint or any other conventional hinges known in the art.

Although in the present embodiment of the invention the lithotripter is provided with three light emitters, other embodiments of the inventive monitoring system may comprise only two light emitters provided on the lithotripter 1 corresponding to two different positions of the C-arc 2, or comprise even more than three light emitters, for example, four, five or more corresponding to four, five or more different positions of the C-arc 2.

The positioning marker 14 is placed on the inner surface 17 of the upper half 9 of the C-arc. In most applications, the upper half of the C-arc connects the swiveling joint 8 and the image intensifier 6, as shown in the present embodiment. However, in other embodiments of the invention, depending on the application, the upper half 9 of the C-arc 2 may also be defined as the part of the C-arc connecting the swiveling joint 8 with the x-ray source 5. Some x-ray C-arcs can be tilted 180° about the tilting axis 7, thereby inverting the C-arc so that the x-ray source 5 takes the place of the image intensifier 6 and vice versa, in order to adapt the C-arc to different applications. In this case, the C-arc can also be provided with two positioning markers, one placed on the upper half 9 and one on the lower half 10 of the C-arc. Favourably, both positioning markers correspond to each of the light emitters 11, 12, 13.

In the present embodiment of the monitoring system, the positioning marker 14 is provided with a mark, namely a target crosshair 18. This facilitates observing the correct alignment. Further, an additional misalignment marker (23), e.g. a circle, can optionally be provided on the position marker 14, marking the limits for the light beam to show a possible misalignment, thus specifying tolerance limits of misalignment. However, in other embodiments of the invention, the mark and marker may differ from the designs described above. Alternatively, it is also possible to provide the position marker 14 solely with the marker.

In the following, the mode of operation of the inventive embodiment shown in the figures will be described.

Before therapy is started, the C-arc 2 and the lithotripter 1 are aligned with each other according to common adjustment methods, for example, by using a focus dummy for the focus point of the lithotripter 1. In this instance, the lithotripter 1 and the C-arc 2 are ideally placed perpendicular to each other, thereby spanning the spatial coordinate system (22) shown in Figure 1. However, in most applications also an approximately perpendicular arrangement will do. When the C-arc 2 is aligned with the lithotripter 1, the light emitters 11, 12, 13 are adjusted such that the hitting point of the light beams generated by the light emitters align with the center of the target crosshairs 18 and lies within circle (23), when the C-arc 2 takes different predetermined positions respectively. Therefore, the C-arc 2 is moved into a first predetermined position. In the present embodiment, this first predetermined position is the upright position, namely the PA position of the C-arc, shown in Figure 1. Then, the first light emitter 12 is adjusted such that the light beam generated by it hits the positioning marker 14. This adjustment may be affected either manually or motorized, for example, by a remote control via a computer system. As in the present embodiment of the invention the positioning marker 14 is additionally provided with target crosshairs 18, the light emitter 12 is adjusted such that the light beam hits the centre of the target crosshairs when the C-arc 2 is in the PA position.

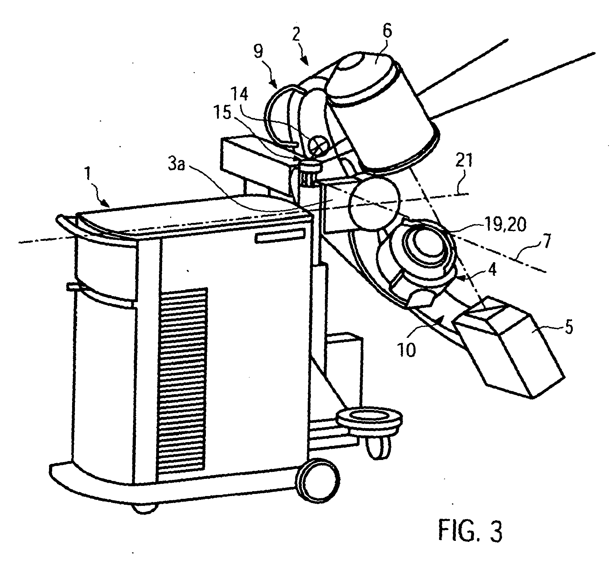

Next, the C-arc 2 is moved to a second predetermined position. In the present embodiment, the C-arc 2 is tilted about tilting axis 7 counterclockwise to a CC- position, as shown in Figure 3. Then, the second light emitter 13 is adjusted such that its light beam hits the centre of the target crosshairs 18 of the positioning marker 14.

Advantageously, the first light emitter 12 may be switched off for doing the adjustment of the second light emitter in order to avoid irritation by the light beam of the first light emitter 12.

In some embodiments of the invention, which only provide two light emitters, or in applications in which the use of only two predetermined positions of the C-arc is sufficient, the set up process will end here. Nevertheless, in the present embodiment, the C-arc 2 is additionally moved to a third predetermined position by moving the C-arc 2 into a CC+ position, as shown in Figure 5. Then, the third light emitter 11 is adjusted such that its light beam hits the centre of the target crosshairs 18 of the positioning marker 14.

Hence, all the light emitters 11, 12, 13 of the present embodiment of the inventive monitoring system are adjusted to correspond to three different positions of the C-arc 2 respectively. Now, therapy, for example a shock wave therapy using the lithotripter 1, can be started.

By means of the light emitters 11, 12, 13 and their generated light beams, it is possible to monitor the correct alignment of the C-arc 2 and the lithotripter 1 during therapy in all three predetermined positions, for example, during the location of a concrement in a patient's body. This usually requires taking x-ray pictures of the patient in at least two different positions of the C-arc. Thus, the first x-ray picture can be taken, for example, in the first predetermined position of the C-arc 2, shown in Figure 1, thereby monitoring the correct alignment of the C-arc 2 and the lithotripter 1 by checking the position of the hitting point of the light beam generated by the first light emitter 12 on the positioning marker 14.

Then, the C-arc 2 is moved to a second predetermined position shown in Figure 3 to take the second x-ray picture, thereby also monitoring the correct alignment of the C-arc 2 and the lithotripter 1 by checking the position of the hitting point of the light beam generated by the second light emitter 13 on the positioning marker 14.

In the present embodiment, even a third x-ray picture can be taken from a third predetermined position of the C-arc 2, thereby also monitoring the correct alignment of the C-arc 2 and the lithotripter 1 by checking the position of the hitting point of the light beam generated by the third light emitter 11 on the positioning marker 14.

From the x-ray pictures taken from different directions, the position of the concrement in the patient's body is derived and the patient table (not shown) is moved such that the concrement aligns with the isocenter 19 of the C-arc 2 and the focus 20 of the lithotripter 1. Next, shock waves are introduced into the patient's body. Also during these steps, the correct alignment of the lithotripter and the C-arc can be monitored by means of the hitting point of the light beam of the third light emitter 11.

During therapy, it may be necessary to move the therapy head 4 to different positions in order to hit the concrement by the shock waves from different positions. Due to the special mechanics of the arm 3, representing a compulsory guide for the therapy head 4, the focus point 20 of the lithotripter 1 always stays in place even when the therapy head 4 or arm 3 is moved. Also, during this process the correct alignment of the lithotripter 1 and the C-arc 2 can be monitored by means of the hitting point of the light beam generated by the third light emitter 11.

Due to the above-described favourable arrangement of the light emitters 11, 12, 13, the light beam of a light emitter extends at a predetermined angle α away from the support arm 3a and thus away from the operation area of the therapy head 4. It is therefore not interrupted by any parts of the lithotripter, even when the therapy head 4 is moved to its highest therapy position. Concurrently, the light beams of the present embodiment extend in a height that is lower than the average height of a human head of the medical personnel standing by. This helps to prevent contact of the light beam with the eyes of the medical personnel and thus helps to avoid irritation by the light beams.

Since additionally each light emitter can be controlled separately, independently from the other light emitters, it is possible to energize only one light emitter at one time, namely the particular light emitter needed for monitoring the correct alignment of the lithotripter 1 and the C-arc 2 in the present situation and to switch off the light emitters and thus the light beams not needed at the moment. If the monitoring system is not needed, e.g. if the concrement is located by different means than x-ray, it is also possible to switch off all light emitters 11, 12, 13. This also helps to prevent glare and irritation of medical personnel.

On the other hand, the present embodiment of the monitoring system also allows switching on all light emitters, so that all light beams are present at one time. This is helpful if, for example, the x-ray procedure is observed and controlled by the medical personnel from a separate control room being different from the examination room. In an alternative embodiment of the inventive monitoring system, the light emitters 11, 12, 13 may be controlled via a remote control.

The target crosshairs 18 on the positioning marker 14 ease the observation of the correct alignment and allow the recognition of the direction of a possible misalignment. The crosshairs can additionally be provided with a scale or other additional misalignment marker (23), e.g. a circle, thereby allowing the determination of the value of misalignment. Depending on the specific embodiment of the lithotripter and the C-arc as well as of the monitoring system, this may also permit a realignment of the C-arc 2 and the lithotripter 1. Therefore, the lithotripter 1 and/or the C-arc 2 are moved such that the light beams generated by the light emitters 11, 12, 13 hit the position marker 14 and in particular a center of the target crosshairs 18. If an additional misalignment marker 23, like a circle, is provided on the positioning marker 14, the lithotripter and/or C-arc are moved such that hitting points of the light beams lay within the misalignment marker 23.

However, in other embodiments of the invention, the positioning marker 14 does not necessarily have to be provided with target crosshairs. Monitoring the correct alignment can also be performed using a blank positioning marker. In this case, the size of the positioning marker can be adapted to the diameter of the light beams. Alternatively, the positioning marker can be provided with any other kind of mark, like a dot or a scale.

The favourable height at which the positioning marker 14 is mounted on the C-arc 2, and the fact that only one positioning marker 14 has to be observed for all three positions of the C-arc 2 further facilitates checking the correct alignment of the lithotripter 1 and the C-arc 2.

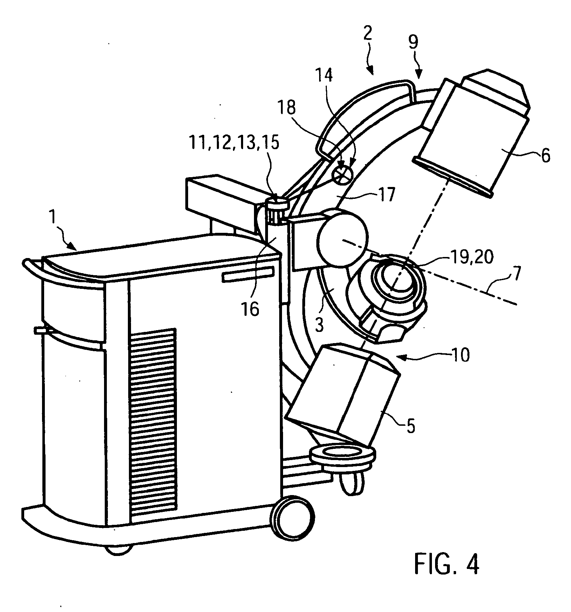

The inventive system not only allows the monitoring of the correct alignment while the therapy head 4 of the lithotripter can be moved arbitrarily during therapy, but also allows moving the C-arc 2 during therapy between predetermined positions. Thus it is, for example, possible to relocate a concrement during therapy, e.g. when the patient has been repositioned or in order to check if the position of the patient is still correct. Therefore, the above-described x-ray procedure is repeated. The C-arc is moved to the predetermined positions shown in Figures 1, 3 and 4 and x-ray pictures are taken in each of the positions as described above. Thereby the correct alignment of the lithotripter and the C-arc is monitored by checking that the light beam of the first light emitter 12 hits the centre of the target crosshairs 18 on the positioning marker 14 when the C-arc 2 is in the first predetermined position, as shown in Figure 1, the light beam of the second light emitter 13 hits the centre of the target crosshairs 18 on the positioning marker 14 when the C-arc 2 is in the second predetermined position, as shown in Figure 3, and the light beam of the third light emitter 11 hits the centre of the target crosshairs 18 on the positioning marker 14 when the C-arc 2 is in the third predetermined position, as shown in Figure 4.

Thus, the inventive system permits the monitoring of the correct alignment of the C-arc 2 and the lithotripter 1 while concurrently allowing movement of the C-arc 2 and the therapy head 4 of the lithotripter.

## Claims

1. Monitoring System for monitoring the adjustment of a lithotripter (1) and a x-ray C-arc (2) during therapy, in which the C-arc (2) is provided with at least one positioning marker (14) and the lithotripter (1) is provided with at least two light emitters (11, 12) and a light beam generated by each of the light emitters (11, 12) hits the positioning marker (14) when the correct adjustment of the lithotripter (1) and the C-arc (2) is maintained,
**characterized in that**,
the different light emitters (11, 12, 13) correspond to different positions of the C-arc (2).

2. System according to claim 1,
**characterized in that**,
the different light emitters (11, 12, 13) correspond to different tilting positions of the C-arc (2).

3. System according to at least one of the preceding claims,
**characterized in that**,
each light emitter (11, 12, 13) is separately hinged.

4. System according to at least one of the preceding claims,
**characterized in that**,
each light emitter (11, 12. 13) is hinged by a magnetic ball joint mounting.

5. System according to at least one of the preceding claims,
**characterized in that**,
the light emitters (11, 12, 13) are adjustable independently from each other.

6. System according to at least one of the preceding claims,
**characterized in that**,
each light emitter (11, 12, 13) can be controlled separately.

7. System according to at least one of the preceding claims,
**characterized in that**, the light emitters (11, 12, 13) are adjustable manually and/or via at least one drive motor.

8. System according to at least one of the preceding claims,
**characterized in that**,
the light emitters (11, 12, 13) are controllable via a remote control.

9. System according to at least one of the preceding claims, **characterized in that**,
the light emitters (11, 12, 13) are positioned on a static part (16) of the lithotripter (2).

10. System according to at least one of the preceding claims,
**characterized in that**,
the light emitters (11, 12, 13) are positioned in a height being higher than a maximum height of a support arm (3a) carrying a therapy arm (3) of the lithotripter (1).

11. System according to at least one of the preceding claims,
**characterized in that**,
the light beams generated by the emitters (11, 12, 13) are slanted versus the spatial coordinates (X, Y, Z - axis) spanned by the arrangement of the lithotripter (1) and the C-arc (2).

12. System according to at least one of the preceding claims,
**characterized in that**,
the light beams generated by the emitters (11, 12, 13) are slanted versus the floor on which the lithotripter (1) stands.

13. System according to at least one of the preceding claims,
**characterized in that**,
at least three light emitters (11, 12, 13) are provided corresponding to three different positions of the C-arc (2), in particular one light emitter (11) corresponding to the PA position of the C-arc, one (12) to the CC+ and one (13) to the CC- position of the C-arc (2).

14. System according to at least one of the preceding claims,
**characterized in that**,
the positioning marker (14) is located on the upper half (9) of the C-arc (2).

15. System according to at least one of the preceding claims,
**characterized in that**,
only one positioning marker (14) is provided matching each of the light beams (11, 12, 13).

16. System according to at least one of the preceding claims,
**characterized in that**,
the positioning marker (14) comprises cross hairs (18).

17. System according to at least one of the preceding claims,
**characterized in that**
the positioning marker (14) comprises a marker (23), in particular a circle, indicating the tolerance limits of misalignment.

18. System according to claim 16,
**characterized in that**,
the light beam hits the center of the cross hairs (18) when a correct adjustment of the lithotripter (1) to the C-arc (2) is maintained.

19. System according to at least one of the preceding claims,
**characterized in that**,
a therapy head (4) and therapy arm (3) of the lithotripter (1) are moveable independently from the light emitters (11, 12, 13).

20. Method for monitoring the adjustment of a lithotripter (1) and a x-ray C-arc (2) during therapy, in which two light beams are generated by at least two light emitters (11, 12, 13) on the lithotripter (1) and each of the light beams hits at least one positioning marker (14) on the C-arc (2), when correct adjustment of the lithotripter (1) and the C-arc (2) is maintained,
**characterized in that**,
each light beam corresponds to a different position of the C-arc (2).

21. Method according to claim 20,
**characterized in that**,
each light emitter (11, 12, 13) is adjusted such, that the corresponding light beam hits the positioning marker (14) at a predetermined tilting position of the C-arc (2).

22. Method according to at least one of claims 20 to 21,
**characterized in that**,
the light emitters (11, 12, 13) are adjusted such, that the light beam of the first light emitter (11) hits the positioning marker (14) when the C-arc (2) is in PA position and the light beam of the second light emitter (12) hits the positioning marker (14) when the C-arc (2) is in one of a CC+ and a CC- position.

23. Method according to claim 22,
**characterized in that**,
a third light emitter (13) is adjusted such, that the corresponding light beam hits the positioning marker (14) when the C-arc (2) is in the other of the CC+ and CC- positions.

24. Method according to at least one of the claims 20 to 23,
**characterized in that**,
only one light emitter (11, 12, 13) is energized at one time.

25. Method according to at least one of the claims 20 to 24,
**characterized in that**,
when the C-arc (2) is in a predetermined position only the light emitter (11, 12, 13) corresponding to this position is energized.

26. Method according to at least one of the claims 20 to 25,
**characterized in that**
all light emitters (11, 12, 13) can be turned off.

27. Method according to at least one of the claims 20 to 26,
**characterized in that**
all light emitters (11, 12, 13) can be turned on.

28. Method according to at least one of the claim 20 to 27,
**characterized in that**,
a therapy head (4) and therapy arm (3) of the lithotripter (1) are moved independently from the light emitters (11, 12, 13).

29. Method according to at least one of the claims 20 to 28,
**characterized in that**,
in case of misalignment, the lithotripter (1) and the C-arc (2) are readjusted by movement of the lithotripter (1) and/or the C-arc (2) such that the light beams generated by the light emitters (11, 12, 13) hit the positioning marker (14), in particular the center of crosshairs (18) within the misalignment marker (23).

## Patentansprüche

1. Überwachungssystem zum Überwachen der Einstellung eines Nierensteinzertrümmerers (1) und eines Röntgen-C-Bogens (2) während der Behandlung, wobei der C-Bogen (2) mit wenigstens einer Positioniermarkierung (14) versehen ist und der Nierensteinzertrümmerer (1) mit wenigstens zwei Lichtemissionseinrichtungen (11, 12) versehen ist und ein von jeder der Lichtemissionseinrichtungen (11, 12) erzeugter Lichtstrahl auf der Positioniermarkierung (14) auftrifft, wenn die richtige Ausrichtung des Nierensteinzertrümmerers (1) und des C-Bogens (2) aufrechterhalten wird,
**dadurch gekennzeichnet, dass**
die verschiedenen Lichtemissionseinrichtungen (11, 12, 13) verschiedenen Positionen des C-Bogens (2) entsprechen.

2. System nach Anspruch 1,
**dadurch gekennzeichnet, dass**
die verschiedenen Lichtemissionseinrichtungen (11, 12, 13) verschiedenen Neigungspositionen des C-Bogens (2) entsprechen.

3. System nach wenigstens einem der vorangehenden Ansprüche,
**dadurch gekennzeichnet, dass**
jede Lichtemissionseinrichtung (11, 12, 13) separat gelenkig gelagert ist.

4. System nach wenigstens einem der vorangehenden Ansprüche,
**dadurch gekennzeichnet, dass**
jede Lichtemissionseinrichtung (11, 12, 13) mit einer magnetischen Kugelgelenk-Anbringung gelenkig gelagert ist.

5. System nach wenigstens einem der vorangehenden Ansprüche,
**dadurch gekennzeichnet, dass**
die Lichtemissionseinrichtungen (11. 12, 13) unabhängig voneinander eingestellt werden können.

6. System nach wenigstens einem der vorangehenden Ansprüche,
**dadurch gekennzeichnet, dass**
jede Lichtemissionseinrichtung (11, 12, 13) separat gesteuert werden kann.

7. System nach wenigstens einem der vorangehenden Ansprüche,
**dadurch gekennzeichnet, dass** die Lichtemissionseinrichtungen (11, 12, 13) manuell und/oder mittels wenigstens eines Antriebsmotors eingestellt werden können.

8. System nach wenigstens einem der vorangehenden Ansprüche,
**dadurch gekennzeichnet, dass**
die Lichtemissionseinrichtungen (11, 12, 13) über eine Fernsteuerung gesteuert werden können.

9. System nach wenigstens einem der vorangehenden Ansprüche,
**dadurch gekennzeichnet, dass**
die Lichtemissionseinrichtungen (11, 12, 13) an einem statischen Teil (16) des Nierensteinzertrümmerers (2) angeordnet sind.

10. System nach wenigstens einem der vorangehenden Ansprüche,
**dadurch gekennzeichnet, dass**
die Lichtemissionseinrichtungen (11, 12, 13) in einer Höhe angeordnet sind, die höher ist als eine maximale Höhe eines Tragearms (3a), der einen Behandlungsarm (3) des Nierensteinzertrümmerers (1) trägt.

11. System nach wenigstens einem der vorangehenden Ansprüche,
**dadurch gekennzeichnet, dass**
die von den Emissionseinrichtungen (11, 12, 13) erzeugten Lichtstrahlen gegenüber den räumlichen Koordinaten (X, Y, Z - Achse) geneigt sind, die durch die Anordnung aus dem Nierensteinzertrümmerer (1) und dem C-Bogen (2) aufgespannt werden.

12. System nach wenigstens einem der vorangehenden Ansprüche,
**dadurch gekennzeichnet, dass**
die von den Emissionseinrichtungen (11, 12, 13) erzeugten Lichtstrahlen gegenüber dem Boden geneigt sind, auf dem der Nierensteinzertrümmerer (1) steht.

13. System nach wenigstens einem der vorangehenden Ansprüche,
**dadurch gekennzeichnet, dass**
wenigstens drei Lichtemissionseinrichtungen (11, 12, 13) vorhanden sind, die drei verschiedenen Positionen des C-Bogens (2) entsprechen, insbesondere eine Lichtemissionseinrichtung (11), die der PA-Position des C-Bogens entspricht, eine (12), die der CC+-Position entspricht, und eine (13), die der CC--Position des C-Bogens (2) entspricht.

14. System nach wenigstens einem der vorangehenden Ansprüche,
**dadurch gekennzeichnet, dass**
sich die Positioniermarkierung (14) an der oberen Hälfte (9) des C-Bogens (2) befindet.

15. System nach wenigstens einem der vorangehenden Ansprüche,
**dadurch gekennzeichnet, dass**
für jeden der Lichtstrahlen (11, 12, 13) nur eine gemeinsame Positioniermarkierung (14) vorhanden ist.

16. System nach wenigstens einem der vorangehenden Ansprüche,
**dadurch gekennzeichnet, dass**
die Positioniermarkierung (14) ein Fadenkreuz (18) umfasst.

17. System nach wenigstens einem der vorangehenden Ansprüche,
**dadurch gekennzeichnet, dass**
die Positioniermarkierung (14) eine Markierung (23), insbesondere einen Kreis, umfasst, der die Toleranzgrenzen von Fehlausrichtung anzeigt.

18. System nach Anspruch 16,
**dadurch gekennzeichnet, dass**
der Lichtstrahl auf die Mitte des Fadenkreuzes (18) auftrifft, wenn eine richtige Ausrichtung des Nierensteinzertrümmerers (1) zu dem C-Bogen (2) aufrechterhalten wird.

19. System nach wenigstens einem der vorangehenden Ansprüche,
**dadurch gekennzeichnet, dass**
ein Behandlungskopf (4) und ein Behandlungsarm (3) des Nierensteinzertrümmerers (1) unabhängig von den Lichtemissionseinrichtungen (11, 12, 13) bewegt werden können.

20. Verfahren zum Überwachen der Einstellung eines Nierensteinzertrümmerers (1) und eines Röntgen-C-Bogens (2) während der Behandlung, wobei zwei Lichtstrahlen von wenigstens zwei Lichtemissionseinrichtungen (11, 12, 13) an dem Nierensteinzertrümmerer (1) erzeugt werden und jeder der Lichtstrahlen auf wenigstens einer Positioniermarkierung (14) an dem C-Bogen (2) auftrifft, wenn eine korrekte Ausrichtung des Nierensteinzertrümmerers (1) und des C-Bogens (2) aufrechterhalten wird,
**dadurch gekennzeichnet, dass**
jeder Lichtstrahl einer anderen Position des C-Bogens (2) entspricht.

21. Verfahren nach Anspruch 20,
**dadurch gekennzeichnet, dass**
jede Lichtemissionseinrichtung (20) so eingestellt wird, dass der entsprechende Lichtstrahl auf die Positioniermarkierung (14) an einer vorgegebenen Neigungsposition des C-Bogens (2) auftrifft.

22. Verfahren nach wenigstens einem der Ansprüche 20 bis 21,
**dadurch gekennzeichnet, dass**
die Lichtemissionseinrichtungen (11, 12, 13) so eingestellt werden, dass der Lichtstrahl der ersten Lichtemissionseinrichtung (11) auf der Positioniermarkierung (14) auftritt, wenn sich der C-Bogen (2) an der PA-Position befindet, und der Lichtstrahl der zweiten Lichtemissionseinrichtung (12) auf der Positioniermarkierung (14) auftrifft, wenn sich der C-Bogen (2) an einer CC+- bzw. einer CC--Position befindet.

23. Verfahren nach Anspruch 22,
**dadurch gekennzeichnet, dass**
eine dritte Lichtemissionseinrichtung (13) so eingestellt wird, dass der entsprechende Lichtstrahl auf der Positioniermarkierung (14) auftrifft, wenn sich der C-Bogen (2) an der anderen von der CC+- und der CC--Position befindet.

24. Verfahren nach wenigstens einem der Ansprüche 20 bis 23,
**dadurch gekennzeichnet, dass**
jeweils nur eine Lichtemissionseinrichtung (11, 12, 13) zur gleichen Zeit eingeschaltet ist.

25. Verfahren nach wenigstens einem der Ansprüche 20 bis 24,
**dadurch gekennzeichnet, dass**
wenn sich der C-Bogen (2) an einer vorgegebenen Position befindet, nur die Lichtemissionseinrichtung (11, 12, 13) eingeschaltet wird, die dieser Position entspricht.

26. Verfahren nach wenigstens einem der Ansprüche 20 bis 25,
**dadurch gekennzeichnet, dass**
alle Lichtemissionseinrichtungen (11, 12, 13) abgeschaltet werden können.

27. Verfahren nach wenigstens einem der Ansprüche 20 bis 26,
**dadurch gekennzeichnet, dass**
alle Lichtemissionseinrichtungen (11, 12, 13) angeschaltet werden können.

28. Verfahren nach wenigstens einem der Ansprüche 20 bis 27,
**dadurch gekennzeichnet, dass**
ein Behandlungskopf (4) und ein Behandlungsarm (3) des Nierensteinzertrümmerers (1) unabhängig von den Lichtemissionseinrichtungen (11, 12, 13) bewegt werden.

29. Verfahren nach wenigstens einem der Ansprüche 20 bis 28,
**dadurch gekennzeichnet, dass**
bei Fehlausrichtung der Nierensteinzertrümmerer (1) und der C-Bogen (2) durch Bewegung des Nierensteinzertrümmerers (1) und/oder des C-Bogens (2) so neu eingestellt werden, dass die von den Lichtemissionseinrichtungen (11, 12, 13) erzeugten Lichtstrahlen auf der Positioniermarkierung (14), insbesondere auf der Mitte eines Fadenkreuzes (18), innerhalb der Fehlausrichtungs-Markierung (23) auftreffen.

## Revendications

1. Système de surveillance pour surveiller le réglage d'un lithotriteur (1) et d'un arc en C à rayons X (2) au cours d'une thérapie, dans lequel l'arc en C à rayons X (2) est doté d'au moins un repère de positionnement (14) et le lithotriteur (1) est doté d'au moins deux émetteurs de lumière (11, 12) et un faisceau de lumière généré par chacun des émetteurs de lumière (11, 12) vient frapper le repère de positionnement (14) lorsque le réglage correct du lithotriteur (1) et de l'arc en C (2) est maintenu,
**caractérisé en ce que**
les différents émetteurs de lumière (11, 12, 13) correspondent à différentes positions de l'arc en C (2).

2. Système selon la revendication 1,
**caractérisé en ce que**
les différents émetteurs de lumière (11, 12, 13) correspondent à différentes positions d'inclinaison de l'arc en C (2).

3. Système selon l'une au moins des revendications précédentes,
**caractérisé en ce que**
chaque émetteur de lumière (11, 12, 13) est articulé séparément.

4. Système selon l'une au moins des revendications précédentes,
**caractérisé en ce que**
chaque émetteur de lumière (11, 12, 13) est articulé par un support de joint à rotule magnétique.

5. Système selon l'une au moins des revendications précédentes,
**caractérisé en ce que**
les émetteurs de lumière (11, 12, 13) sont réglables indépendamment les uns des autres.

6. Système selon l'une au moins des revendications précédentes,
**caractérisé en ce que**
chaque émetteur de lumière (11, 12, 13) peut être commandé séparément.

7. Système selon l'une au moins des revendications précédentes,
**caractérisé en ce que**
les émetteurs de lumière (11, 12, 13) sont aptes à être réglés manuellement et/ou par l'intermédiaire d'au moins un moteur d'entraînement.

8. Système selon l'une au moins des revendications précédentes,
**caractérisé en ce que**
les émetteurs de lumière (11, 12, 13) sont aptes à être commandés par l'intermédiaire d'une télécommande.

9. Système selon l'une au moins des revendications précédentes,
**caractérisé en ce que**
les émetteurs de lumière (11, 12, 13) sont positionnés sur une partie statique (16) du lithotriteur (1).

10. Système selon l'une au moins des revendications précédentes,
**caractérisé en ce que**
les émetteurs de lumière (11, 12, 13) sont positionnés à une hauteur plus importante qu'une hauteur maximale d'un bras de support (3a) portant un bras de thérapie (3) du lithotriteur (1).

11. Système selon l'une au moins des revendications précédentes,
**caractérisé en ce que**
les faisceaux de lumière générés par les émetteurs (11, 12, 13) sont inclinés par rapport aux coordonnées spatiales (axes X, Y, Z) couvertes par la disposition du lithotriteur (1) et de l'arc en C (2).

12. Système selon l'une au moins des revendications précédentes,
**caractérisé en ce que**
les faisceaux de lumière générés par les émetteurs (11, 12, 13) sont inclinés par rapport au sol sur lequel repose le lithotriteur (1).

13. Système selon l'une au moins des revendications précédentes,
**caractérisé en ce que**
au moins trois émetteurs de lumière (11, 12, 13) sont disposés de façon à correspondre à trois positions différentes de l'arc en C (2), notamment de façon à ce qu'un émetteur de lumière (11) corresponde à la position PA de l'arc en C, un émetteur (12) à la position CC+ et un émetteur (13) à la position CC- de l'arc en C (2).

14. Système selon l'une au moins des revendications précédentes,
**caractérisé en ce que**
le repère de positionnement (14) est situé sur la moitié supérieure (9) de l'arc en C (2).

15. Système selon l'une au moins des revendications précédentes,
**caractérisé en ce que**
seul un repère de positionnement (14) est disposé de façon à correspondre à chacun des faisceaux de lumière (11, 12, 13).

16. Système selon l'une au moins des revendications précédentes,
**caractérisé en ce que**
le repère de positionnement (14) comprend des fils croisés (18).

17. Système selon l'une au moins des revendications précédentes,
**caractérisé en ce que**
le repère de positionnement (14) comprend un repère (23), notamment un cercle, indiquant les limites de tolérance de défaut d'alignement.

18. Système selon la revendication 16,
**caractérisé en ce que**
le faisceau lumineux frappe le centre des fils croisés (18) lorsqu'un réglage correct du lithotriteur (1) par rapport à l'arc en C (2) est maintenu.

19. Système selon l'une au moins des revendications précédentes,
**caractérisé en ce que**
une tête de thérapie (4) et un bras de thérapie (3) du lithotriteur (1) sont aptes à être déplacés indépendamment des émetteurs de lumière (11, 12, 13).

20. Procédé pour surveiller le réglage d'un lithotriteur (1) et d'un arc en C à rayons X (2) au cours d'une thérapie, dans lequel deux faisceaux de lumière sont générés par au moins deux émetteurs de lumière (11, 12, 13) sur le lithotriteur (1) et chacun des faisceaux de lumière vient frapper au moins un repère de positionnement (14) sur l'arc en C (2) lorsque le réglage correct du lithotriteur (1) et de l'arc en C (2) est maintenu,
**caractérisé en ce que**
chaque faisceau de lumière correspond à une position différente de l'arc en C (2).

21. Procédé selon la revendication 20,
**caractérisé en ce que**
chaque émetteur de lumière (11, 12, 13) est réglé de façon à ce que le faisceau de lumière correspondant vienne frapper le repère de positionnement (14) au niveau d'une position d'inclinaison prédéterminée de l'arc en C (2).

22. Procédé selon l'une au moins des revendications 20 et 21,
**caractérisé en ce que**
les émetteurs de lumière (11, 12, 13) sont réglés de telle sorte que le faisceau lumineux du premier émetteur de lumière (11) vienne frapper le repère de positionnement (14) lorsque l'arc en C (2) est dans la position PA et que le faisceau lumineux du second émetteur de lumière (12) vienne frapper le repère de positionnement (14) lorsque l'arc en C (2) est dans l'une des positions CC+ et CC-.

23. Procédé selon la revendication 22,
**caractérisé en ce que**
un troisième émetteur de lumière (13) est réglé de telle sorte que le faisceau de lumière correspondant vienne frapper le repère de positionnement (14) lorsque l'arc en C (2) est dans l'autre des positions CC+ et CC-.

24. Procédé selon l'une au moins des revendications 20 à 23,
**caractérisé en ce que**
un seul émetteur de lumière (11, 12, 13) à la fois est excité.

25. Procédé selon l'une au moins des revendications 20 à 24,
**caractérisé en ce que**
lorsque l'arc en C (2) est dans une position prédéterminée, seul l'émetteur de lumière (11, 12, 13) correspondant à cette position est excité.

26. Procédé selon l'une au moins des revendications 20 à 25,
**caractérisé en ce que**
tous les émetteurs de lumière (11, 12, 13) peuvent être désactivés.

27. Procédé selon l'une au moins des revendications 20 à 26,
**caractérisé en ce que**
tous les émetteurs de lumière (11, 12, 13) peuvent être activés.

28. Procédé selon l'une au moins des revendications 20 à 27,
**caractérisé en ce que**
une tête de thérapie (4) et un bras de thérapie (3) du lithotriteur (1) sont déplacés indépendamment des émetteurs de lumière (11, 12, 13).

29. Procédé selon l'une au moins des revendications 20 à 28,
**caractérisé en ce que**
en cas de défaut d'alignement, le lithotriteur (1) et l'arc en C (2) sont re-réglés par déplacement du lithotriteur (1) et/ou de l'arc en C (2) de telle manière que les faisceaux de lumière générés par les émetteurs de lumière (11, 12, 13) viennent frapper le repère de positionnement (14), notamment le centre des fils croisés (18) à l'intérieur du repère de défaut d'alignement (23).
